# EUROPÄISCHE PATENTSCHRIFT

(11) **EP 2 796 100 B1**
(45) Veröffentlichungstag und Bekanntmachung des Hinweises auf die Patenterteilung: **20.04.2016**
(21) Anmeldenummer: 13164955.0
(22) Anmeldetag: 23.04.2013
(51) Int. Cl.: A61B 17/22, A61K 47/36, A61L 24/00, A61L 24/04

(54) **Gelbildendes System zum Entfernen von Nierensteinfragmenten**
Gelling system for the removal of kidney stone fragments
Système gélifiant pour éliminer des fragments de calculs rénaux

(43) Veröffentlichungstag der Anmeldung: 29.10.2014
(73) Patentinhaber: Fraunhofer-Gesellschaft zur Förderung der angewandten Forschung e.V., 80686 München (DE); Albert-Ludwigs-Universität Freiburg, 79085 Freiburg (DE)
(72) Erfinder: Grunwald, Ingo, Dr., 28865 Lilienthal (DE); Richter, Katharina, Dr., 28195 Bremen (DE); Miernik, Arkadiusz, Dr., 79104 Freiburg (DE); Schoenthaler, Martin, Dr., 79100 Freiburg (DE)
(74) Vertreter: Eisenführ Speiser

(56) Entgegenhaltungen:
- WO-A2-2004/080343
- WO-A2-2009/070766
- CA-A1- 2 710 986
- US-A1- 2002 119 116
- US-A1- 2012 108 676
- P.-E. LE RENARD ET AL.: "The in vivo performance of magnetic particle-loaded injectible, in situ gelling, carriers for the delivery of local hyperthermia", BIOMATERIALS, Bd. 31, 2010, Seiten 691-705, XP002712366,
- ANONYMOUS: 'Medical Devices: Guidance document', [Online] 03 Dezember 2009, XP055023202 Gefunden im Internet: <URL:http://ec.europa.eu/health/medical-dev ices/files/meddev/2_1_3_rev_3-12_2009_en.pd f> [gefunden am 2012-03-28]

## Beschreibung

Die vorliegende Erfindung betrifft primär eine gelbildende Zusammensetzung zur Anwendung in einem Verfahren zum Entfernen von Nierensteinen und/oder Nierensteinfragmenten aus dem Körper.

Insbesondere betrifft die vorliegende Erfindung eine gelbildendende Zusammensetzung zur Anwendung in einem hierin beschriebenen Verfahren, die Zusammensetzung bestehend aus oder umfassend eine Zusammensetzung (A), enthaltend ein oder mehrere kationisch vernetzbare Polymere, und eine Zusammensetzung (B), enthaltend ein oder mehrere Vernetzungsmittel zum Vernetzen des bzw. der kationisch vernetzbaren Polymere, so dass bei Kontakt der Zusammensetzung (A) mit der Zusammensetzung (B) in einem Bereich der Niere, der Nierensteine und/oder Nierensteinfragmente enthält, ein vernetztes Gel entsteht, das die zu entfernenden Nierensteine und/oder Nierensteinfragmente teilweise oder vollständig umschließt. Weiterhin betrifft die vorliegende Erfindung die Anwendung einer gelbildenden Zusammensetzung in einem hierin beschriebenen Verfahren, bestehend aus oder umfassend die Zusammensetzungen (A) und (B) wie hierin beschrieben, sowie zudem umfassend magnetisierbare Teilchen, zum teilweisen oder vollständigen Umschließen von Nierensteinen und/oder Nierensteinfragmenten durch Ausbilden eines vernetzten Gels bei Kontakt der Zusammensetzung (A) mit der Zusammensetzung (B), wobei das vernetzte Gel die magnetisierbaren Teilchen enthält.

Die vorliegende Erfindung betrifft demnach auch ein vernetztes Gel zur Anwendung in einem hierin beschriebenen Verfahren zum teilweisen oder vollständigen Umschließen von Nierensteinen und/oder Nierensteinfragmenten, wobei das vernetzte Gel magnetisierbare Teilchen enthält und herstellbar oder hergestellt ist durch Bereitstellung einer Zusammensetzung (A) und (B) wie hierin beschrieben, sowie optional einer Zusammensetzung (C), wobei die Zusammensetzung (A) und/oder die Zusammensetzung (B) und/oder die Zusammensetzung (C) magnetisierbare Teilchen enthält, und in-Kontakt-Bringen der Zusammensetzungen (A) und (B) (sowie gegebenenfalls (C)) unter Bedingungen, die eine Vernetzung des bzw. der kationisch vernetzbaren Polymere ermöglichen, so dass ein vernetztes Gel entsteht.

Weitere Aspekte der vorliegenden Erfindung ergeben sich aus der nachfolgenden Beschreibung, insbesondere den Beispielen, sowie den beigefügten Patentansprüchen.

Nierensteine, medizinisch als Nephrolithen bezeichnet, sind Ablagerungen ausgefallener Salze in den Nierengängen oder ableitenden Harnwegen (Harnsteine), die dort häufig starke Schmerzen (Nierenkolik) verursachen. In der überwiegenden Zahl der Fälle handelt es sich um Kalziumoxalatsteine. Daneben kommen aber auch Harnsäuresteine (Urate), Magnesiumammoniumphosphatsteine und Calciumphosphatsteine, sowie weitere mit geringerer Häufigkeit auftretende Zusammensetzungen vor.

Etwa 30 Millionen Menschen in Europa leiden unter Nierensteinen (ca. 5% der Bevölkerung), und die Häufigkeit von Harnsteinerkrankungen in den industrialisierten Ländern zeigt eine ansteigende Tendenz. Besonders hoch (etwa 60%) ist das Risiko, nach Heilung wiederholt von Nierensteinen betroffen zu sein. Medizinische Komplikationen, die im Zusammenhang mit Nierensteinen auftreten können, sind Nierenfunktionsverlust und Infektionskomplikationen bis hin zur Blutvergiftung. Die entstehenden Belastungen für die Gesundheitssysteme sind gravierend.

Kleine Nierensteine wandern häufig von selbst über den Harnleiter in die Harnblase. Erfolgt kein spontaner Abgang, müssen die Steine mit Hilfe anderer Techniken entfernt werden.

Eine Möglichkeit, die Position oder Verteilung von Substanzen oder Objekten im Körper gezielt zu steuern, ist die Ausnutzung magnetischer Wechselwirkungen. Dazu müssen die Zielsubstanzen bzw. Zielobjekte entsprechend magnetisiert werden. Magnetische (Nano-)Partikel haben sich dazu bereits in verschiedenen biomedizinischen Anwendungen als geeignet erwiesen, da sie eine hohe Biokompabilität aufweisen und mit verschiedenen funktionellen Gruppen modifiziert werden können. So werden magnetische Partikel beispielsweise verwendet, um Wirkstoffe im Körper an ihren gewünschten Wirkort zu transportieren. Therapeutische und diagnostische Substanzen können somit effizient eingesetzt werden, und Schäden durch eventuelle Nebenwirkungen in gesunden Geweben werden minimiert. US 2007/0231393 beschreibt in diesem Zusammenhang ein Verfahren, bei dem magnetische Wirkstoffträger-Partikel mittels eines äußeren magnetischen Feldes im Körper positioniert werden.

US 2009/0136594 beschäftigt sich mit einer Methode, biologische Partikel zu magnetisieren, indem sie mit magnetischen Partikeln in Kontakt gebracht werden, die so modifiziert sind, dass sie spezifisch an die biologischen Partikel binden können. Als eine mögliche Anwendung des beschriebenen Verfahrens können Nierensteine oder deren Fragmente magnetisiert werden, um sie mittels einer Vorrichtung, die solche Partikel magnetisch anzieht, aus dem Körper zu entfernen. Um Kalzium-basierte Biomineralien (wie z.B. Nierensteine) spezifisch zu binden, werden die Partikel mit bestimmten Kalzium-bindenden Proteinen oder Proteinfragmenten modifiziert.

Größere Steine können in der Regel nicht durch einen minimal-invasiven Eingriff entfernt werden und müssen daher zunächst in kleinere Bruchstücke zertrümmert bzw. ganz oder zumindest teilweise aufgelöst werden. Eine Methode zur Behandlung von Nierensteinen durch gezieltes Auflösen der Ablagerungen unter Verwendung von quartären Ammoniumsalzen wird beispielsweise in US 5,244,913 beschrieben.

Eine weitere Möglichkeit der Behandlung von Nierensteinen ohne vorhergehendes Zertrümmern wird in US 2006/0269512 angegeben. Hierbei wird die natürliche Peristaltik dazu ausgenutzt, einen Polymerpfropfen durch ein Lumen zu pressen, und dabei den Stein aus dem Lumen zu entfernen. Der Polymerpfropfen kann in situ durch Temperatur- oder pH-Änderung oder durch ionische Wechselwirkungen gebildet werden.

Bei der Lithotripsie werden Nierensteine durch extrakorporale Stoßwellen oder endoskopisch eingeführte Laser- oder Druckluftsonden zertrümmert. Dabei entstehen unterschiedlich große Bruchstücke, die entweder mit Hilfe von Greifwerkzeugen entfernt oder ausgespült werden können. Ein bei der Lithotripsie auftretendes Problem ist, dass sich die Fragmente während des Zertrümmerns verteilen und dabei umgebendes Gewebe schädigen können oder in schwer zugängliche Regionen gelangen.

WO 2005/037062 betrifft eine Methode, mit der Nierensteine mit Hilfe eines Polymerpfropfens in einem bestimmten Bereich eingeschlossen (nicht umschlossen) werden, wodurch Gewebeschäden durch die entstehenden Fragmente während der Zertrümmerung weitgehend verhindert werden können. Gemäß WO 2005/037062 wird auf zumindest einer Seite eines Nierensteins eine gelbildende Flüssigkeit in das Lumen injiziert, beispielsweise ein thermosensitives Polymer, welches bei Körpertemperatur einen Gelpfropfen bildet. Das Polymer kommt dabei in der Regel nicht in Kontakt mit dem Nierenstein, dient aber dazu, die Effizienz der Lithotripsie zu erhöhen, indem es ein Verschieben des Nierensteins verhindert, und das umgebende Gewebe vor Schädigung durch die Fragmentierung schützt.

Gemäß US 2008/0103481 wird ein biokompatibler Polymerpfropfen insbesondere dazu verwendet, um ein Rückwärtsverschieben von Nierensteinen oder Nierensteinfragmenten während der Lithotripsie zu verhindern und dadurch die Schädigung des umgebenden Gewebes zu minimieren.

Ein Ansatz um Objekte, wie beispielsweise Blutgerinnsel, unter Verwendung eines Klebstoffes aus dem Körper zu entfernen, wird in US 2008/0065012 angegeben. Der Klebstoff wird dabei auf einer Oberfläche verteilt und mit Hilfe eines Katheters in den Körper eingeführt. Wenn das Objekt an der Oberfläche angehaftet ist, wird der Katheter wieder herausgezogen und nimmt das Objekt mit.

Auf biologischen Makromolekülen basierende Klebstoffe und insbesondere gelbildende Polymer-Systeme finden in der Medizintechnik zunehmend Anwendungen. Dabei ist ihre hohe Biokompatibilität eines der wichtigsten Auswahlkriterien.

Thermosensitive oder ionisch polymerisierbare Polymere werden beispielsweise verwendet, um den Blutfluss aus verletzen Blutgefäßen zu stoppen. WO 2008/103891 gibt eine Methode an, bei der der Ausfluss von biologischen Flüssigkeiten aus Gewebe oder Gefäßen durch in situ Bildung eines Polymerpfropfens kontrolliert werden kann.

WO 010544 betrifft einen adhäsiven Proteinschaum und dessen Verwendung für chirurgische und therapeutische Anwendungen. Der Schaum besteht aus einer flüssigen Proteinmatrix und einem biokompatiblen Gas und dient dazu, verletztes Gewebe abzudecken bzw. zu schützen oder implantiertes Gewebe mit biologischem Gewebe zu verbinden.

WO 02/18448 beschreibt die pharmazeutische Verwendung von percarboxylierten Polysacchariden bei der Herstellung von Biomaterialien für chirurgische und biomedizinische Anwendungen. Solche Materialien sind besonders geeignet für den Einsatz im Körper, da sie als körpereigen erkannt werden und keine Immunabstoßungsreaktionen hervorrufen. Sie können daher als Beschichtung für Implantate verwendet werden.

Eine Methode zur Verkapselung von Nierengewebe in Kugeln aus biokompatiblen Polymeren wird in US 2009/0162411 beschrieben. Ziel einer solchen Verkapselung ist es, Nierengewebs-Implantate zu erhalten, die einem Patienten, der unter einer Störung der Nierenfunktion leidet, injiziert werden können, um die Funktion der Niere zu unterstützen.

Kalziumalginat als biokompatibles Hyrdogel-Polymer zum Schließen von Schädelöffnungen nach einer Operation am offenen Gehirn wird in WO 2004/080343 offenbart.

Die Eignung von Polysaccharid-haltigen Polymeren für die Bindung biologisch aktiver Moleküle oder ganzer Zellen im Bereich der Organtransplantation und des künstlichen Gewebeersatzes wird in WO 1998/012228 beschrieben.

Alginate werden im medizinischen und kosmetischen Bereich auch als Füllstoffe für die Unterstützung von Haut und Muskeln verwendet. In US 2011/0097367 werden Anwendungen beschrieben, bei denen monolithische Alginat-Implantate durch Injektion einer reinen hochmolekularen Alginat-Lösung ins Gewebe und spontane Vernetzung in situ gebildet werden. Die Vernetzung erfolgt durch Ca²⁺-Ionenbrücken ohne dass zusätzlich Vernetzungsmittel hinzugegeben werden müssen. Die beschriebenen Alginat-Implantate eignen sich für die Behandlung von Falten oder verschiedenen Krankheiten, bei denen die Muskelstruktur geschwächt ist.

In US 6,663,594 B2 wird ein Verfahren zur Immobilisation eines Objekts, beispielweise eines Nierensteins, im Körper beschrieben, bei dem eine gelbildende Flüssigkeit in den Körper injiziert wird. In Kontakt mit dem Objekt wird ein Gel gebildet, welches das Objekt zumindest teilweise erfasst und immobilisiert. Die Immobilisierung dient dazu, das Objekt anschließend fragmentieren zu können ohne eine Verteilung der Bruchstücke zu riskieren bzw. um das Objekt bzw. die Bruchstücke mit einem endoskopischen Werkzeug aus dem Körper zu entfernen. Dabei verhindert das Gel, dass das Objekt bzw. ein Bruchstück verrutscht und von dem Werkzeug nicht erfasst werden kann. Nach Entfernen des Objekts bzw. der Bruchstücke wird das Gel aufgelöst oder mittel eines endoskopischen Werkzeugs extrahiert. Nachteil dieser Methode ist, dass beim Zertrümmern der Nierensteine das bereits abgebundene Gel zerstört werden kann und dadurch wieder Fragmente freigesetzt werden können, oder dass einzelne Fragmente aus dem Polymer austreten. Zudem ist die beschriebene Prozedur sehr aufwendig, da die Steine oder Stein-Fragmente einzeln erfasst und entfernt werden. Im Ergebnis bleiben mit relativ großer Wahrscheinlichkeit einzelne Steinfragmente zurück.

Ein Problem bei der Lithotripsie ist insbesondere das Auftreten mittelgroßer Steinfragmente (insbesondere < 2 mm), auch als "Gries" bezeichnet, da diese Bruchstücke weder effizient gegriffen noch gespült werden können. Restfragmente dieser Größe rutschen durch die Maschen der Greifinstrumente (Fasszängchen oder -körbchen) und machen die Extraktion von Gries sehr zeitaufwändig und bei größeren Steinmassen praktisch undurchführbar. Bisher wurde noch keine Technologie erfolgreich etabliert, um die mittelgroßen Steinfragmente vollständig zu entfernen. Das Zurückbleiben solcher Nierensteinfragmente führt jedoch unweigerlich zur Bildung neuer Nierensteine, da die Bruchstücke bzw. Fragmente als "Kristallisationskeime" fungieren.

Damit ein vollständiges Entfernen von Fragmenten jeglicher Größe sichergestellt werden kann, muss eine neue Methode entwickelt werden, die dazu geeignet ist, idealerweise alle Bruchstücke zuverlässig zu erfassen. Hierbei sollten vorzugsweise die (zum Teil oben genannten) Probleme und Schwierigkeiten, die im Stand der Technik bekannte Verfahren mit sich bringen, vermieden werden.

Primäre Aufgabe der vorliegenden Erfindung war es, ein System bereitzustellen, das dazu geeignet ist, insbesondere Nierensteinfragmente zuverlässig aus dem Körper extrahieren zu können.

Insbesondere war es Aufgabe der vorliegenden Erfindung, ein gelbildendes System bereitzustellen, das dazu geeignet ist, mittelgroße Nierensteinfragmente zuverlässig aus dem Körper extrahieren zu können.

Weitere Aufgaben der vorliegenden Erfindung ergeben sich aus der nachfolgenden Beschreibung sowie insbesondere den beigefügten Patentansprüchen.

Die primäre Aufgabe wird gemäß einem Aspekt der vorliegenden Erfindung gelöst durch eine gelbildende Zusammensetzung, umfassend eine Zusammensetzung (A), enthaltend ein oder mehrere kationisch vernetzbare Polymere, und eine Zusammensetzung (B), enthaltend ein oder mehrere Vernetzungsmittel zum Vernetzen des bzw. der kationisch vernetzbaren Polymere zur Anwendung in einem Verfahren zur Einbettung und zum Entfernen von Nierensteinen und/oder Nierensteinfragmenten in/aus einem Bereich einer Niere, der zu entfernende Nierensteine und/oder Nierensteinfragmente enthält, mit folgenden Schritten:
(i) Bereitstellen der Zusammensetzungen (A) und (B),
(ii) Einbringen der Zusammensetzungen (A) und (B) in einen Bereich der Niere, der zu entfernende Nierensteine und/oder Nierensteinfragmente enthält,
   unter Bedingungen, die bei Kontakt der Zusammensetzung (A) mit der Zusammensetzung (B) eine Vernetzung des bzw. der kationisch vernetzbaren Polymere ermöglichen, so dass ein vernetztes Gel entsteht, das die zu entfernenden Nierensteine und/oder Nierensteinfragmente teilweise oder vollständig umschließt, wodurch die Entfernung der eingebetteten zu entfernenden Nierensteine und/oder Nierensteinfragmente ermöglicht wird,
(iii) Entfernen des vernetzten Gels samt der davon umschlossenen Nierensteine und/oder Nierensteinfragmente aus der Niere.

Im Rahmen der vorliegenden Erfindung sind unter einem "Bereich der Niere" insbesondere die Nierengänge, aber auch andere Bereiche, in denen Nierensteine auftreten können, sowie die ableitenden Harnwege, Harnleiter, Blase oder Harnröhre zu verstehen.

Unter "Nierensteinfragmenten" sind im Zusammenhang mit der vorliegenden Erfindung Bruchstücke von Nierensteinen zu verstehen, die insbesondere durch Zertrümmerung von Nierensteinen (Lithotripsie) entstanden sind.

Durch ein erfindungsgemäßes Einbetten der Nierensteinfragmente und anschließende Extraktion des "Klebstoff-Verbunds" können vorteilhafterweise Fragmente jeglicher Größe vollständig entfernt und damit einer erneuten Steinbildung vorgebeugt werden.

Die Polymere bzw. Polymer-Einheiten der Zusammensetzung (A) werden bevorzugt über ionische Wechselwirkungen (siehe Zusammensetzung (B)) vernetzt. Daher ist eine Vielzahl von Makromolekülen, die als Liganden ein- oder mehrwertiger Kationen auftreten und Chelatkomplexe bilden können, für die erfindungsgemäße Anwendung geeignet. Dazu gehören insbesondere Hydrogele, biokompatible zuckerbasierte (z.B. modifizierte Cellulosen) oder proteinogene Klebstoffe oder fibrin- oder kollagenbasierte Systeme (besonders bevorzugte Polymere sind weiter unten beschrieben).

Beispielsweise polyphenolische Proteine sind in der Lage, über Quervemetzungen ihres Proteingerüsts durch eine Katecholoxidase abzubinden. In vitro können solche Quervernetzungen z.B. auch durch Metallionen erzielt werden. Denkbar ist auch die Verwendung von Hybridsystemen, die auf einer Kombination von synthetischen Polymeren mit phenolischen Aminosäuren beruhen. Die posttranslationale Aminosäure 3,4-Dihydroxyphenylalanin (DOPA) ist beispielsweise auf Grund ihrer vielseitigen Reaktionsmöglichkeiten mit verschiedenen funktionellen Gruppen zur Polymermodifikation besonders geeignet, und entsprechende Gelsysteme zeichnen sich durch verbesserte adhäsive und kohäsive Eigenschaften aus.

Als Vernetzungsmittel der Zusammensetzung (B) dienen vorzugsweise geeignete Kationen. Vorteilhafterweise handelt es sich dabei in der Regel um natürlich in physiologischen Systemen vorkommende Kationen. Es müssen vorteilhafterweise keine zusätzlichen (aggressiven) Reagenzien zugegeben werden, um die Vernetzung unter physiologischen Bedingungen in Gang zu setzen. Zudem entstehen vorteilhafterweise keine unerwünschten Nebenprodukte.

Erfindungsgemäß bevorzugt sind solcheZusammensetzungen, die unter physiologischen Bedingungen abbinden können. Um stabile Vernetzungen über Kationenbrücken auszubilden, ist es vorteilhaft, wenn die Polymere der Zusammensetzung (A) über funktionelle Gruppen (in ausreichender Anzahl) verfügen, die auch bei (leicht) saurem pH als negativ geladene Einheiten vorliegen. In manchen Zusammensetzungen können beispielsweise der Vernetzungsgrad oder die Geschwindigkeit der Vernetzung über beeinflussbare Größen wie Konzentrationen oder pH-Werte der einzelnen Zusammensetzungen gesteuert werden. Gemäß einer bevorzugten Ausführung enthält die Zusammensetzung (A) und/oder die Zusammensetzung (B) Chitosan. Besonders bevorzugt enthält die Zusammensetzung (B) Chitosan.

Die Zusammensetzungen (A) und (B) können nacheinander oder gemeinsam eingebracht werden, wobei es bevorzugt ist, dass die Zusammensetzung (B) vor der Zusammensetzung (A) eingebracht wird, um eine geeignete Verteilung und vollständige Einbettung aller Nierensteinfragmente vor Beginn bzw. bei der Vernetzung zu gewährleisten.

Das verfestigte Gel hat vorzugsweise eine ausreichende Stabilität und Flexibilität, um anschließend mit möglichst geringem Aufwand, vorzugsweise in einem Stück, aus dem Körper entfernt zu werden.

Eine erfindungsgemäß anzuwendende Zusammensetzung kann zusätzlich weitere Komponenten enthalten. Beispielsweise können den Zusammensetzungen (A) und/oder (B) und/oder einer oder mehreren weiteren Zusammensetzungen einer erfindungsgemäßen Zusammensetzung Substanzen, die die Gelbildung und/oder die Einbindung der Nierensteinfragmente fördern, zugegeben werden. Solche Substanzen können z.B. Quervernetzer zur Erhöhung der Stabilität des Gels sein.

Gemäß einer bevorzugten Ausführung der vorliegenden Erfindung ist bzw. sind das bzw. ein, mehrere oder sämtliche kationisch vernetzbare Polymere der Zusammensetzung (A) ausgewählt aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine.

Polysaccharide wie Alginate und Pektine sind für den Einsatz im Körper besonders geeignet, da sie keine inflammatorischen Reaktionen oder Immunabstoßung hervorrufen und ein geringes Risiko eines Gewebetraumas mit sich bringen. Zudem sind sie biologisch abbaubar und verfügen über zahlreiche Carbonsäuregruppen, die Chelatkomplexe mit mehrwertigen Kationen bilden können. Sie sind vorteilhafterweise dazu in der Lage, unter Wasser und bei physiologischen Temperaturen zu vernetzen und können leicht in Lösung gehandhabt werden. Die Vernetzung erfolgt zügig aber ohne dabei feine Nierenkanälchen oder die Endoskopieinstrumente zu verkleben. Die entstehenden Gele weisen eine ausreichende Stabilität und Flexibilität auf, um zusammen mit den Nierensteinfragmenten extrahiert werden zu können.

Gemäß einer weiteren bevorzugten Ausgestaltung der vorliegenden Erfindung ist bzw. sind das bzw. ein, mehrere oder sämtliche Vernetzungsmittel der Zusammensetzung (B) ausgewählt aus der Gruppe bestehend aus zwei- und dreiwertigen Kationen, vorzugsweise Eisen- und Kalzium-Ionen.

Eisen- und Kalziumionen sind in physiologischen Systemen natürlich vorkommende Kationen, die in Form von biologisch verträglichen Lösungen einfach verabreicht werden können. Sie haben eine geeignete Koordinationschemie und können stabile Chelatkomplexe zur Vernetzung ausbilden.

Gemäß einer weiteren bevorzugten Ausgestaltung der vorliegenden Erfindung weist die Zusammensetzung (B) einen sauren pH-Wert auf, vorzugsweise einen pH im Bereich von 3,5 bis 4,5.

Bei einem pH im Bereich von 3,5 bis 4,5 liegen die Kationen der Zusammensetzung (B) frei in Lösung vor und stehen somit zur Komplexierung zur Verfügung. Vorteilhafterweise sind in diesem pH-Bereich aber die am Polysaccharid befindlichen Säuregruppen zu einem großen Teil deprotoniert, wodurch es zu einer effektiven Vernetzungsreaktion kommt. Wird im Bereich der zu entfernenden Nierensteinfragmente eine gepufferte Lösung (bei einem pH von ca. 4) vorgelegt, kommt es infolge des Einleitens der polysaccharidhaltigen Zusammensetzung (A) zu einer Herabsetzung der Löslichkeit (Koazervation). Der Prozess der Koazervation beansprucht eine gewisse Zeit, während der die Nierensteinfragmente eingebettet werden. Vorteilhafterweise ist damit auch die Geschwindigkeit der Vernetzungsreaktion über den pH der verwendeten Zusammensetzungen steuerbar.

Eine weitere bevorzugte Ausführung der vorliegenden Erfindung betrifft eine gelbildende Zusammensetzung zur Anwendung wie oben beschrieben, wobei die gelbildende Zusammensetzung zudem magnetisierbare Teilchen enthält, wobei
- die magnetisierbaren Teilchen Bestandteil der Zusammensetzung (A) und/oder Bestandteil der Zusammensetzung (B) sind
   und/oder
- ddie gelbildende Zusammensetzung zudem eine Zusammensetzung (C) umfasst, die magnetisierbare Teilchen enthält, wobei dann in Schritt (i) neben den Zusammensetzungen (A) und (B) auch die Zusammensetzung (C) bereitgestellt und in Schritt (ii) zeitversetzt oder gleichzeitig mit der Zusammensetzung (A) oder der Zusammensetzung (B) auch die Zusammensetzung (C) eingebracht wird,
   so dass das vernetzte Gel zudem magnetisierbare Teilchen enthält.

Der Zusatz magnetisierbarer Teilchen eröffnet eine neue und vorteilhafte Methode, den abgebundenen "Klebstoff-Verbund" durch Ausnutzung der magnetischen Eigenschaften aus dem Körper zu entfernen.

Gemäß einer bevorzugten Ausführung der vorliegenden Erfindung sind die magnetisierbaren Teilchen ausgewählt aus Teilchen enthaltend oder bestehend aus ferromagnetische(n) Elemente(n) wie Eisen, Nickel und Kobalt sowie Legierungen wie AlNiCo, SmCo, Nd₂Fe₁₄B, Ni80Fe20, NiFeCo und/oder deren Oxide wie Eisenoxidpartikel, insbesondere Eisenoxidnanopartikel aus Fe₃O₄ und/oder γ-Fe₂O₃.

Eisenoxidpartikel haben sich in medizintechnischen und pharmazeutischen Anwendungen, z.B. als intravenös verabreichtes Kontrastmittel für die Magnetresonanztomographie oder zur Tumortherapie, als geeignet erwiesen. Zur Erhöhung der Biokompatibilität und kolloidalen Stabilität sind solche Partikel meist umhüllt mit z.B. Dextranen, Polyvinylalkoholen, Dimercaptobernsteinsäure u.a..

Die Eisenoxidpartikel verleihen dem Klebstoff außerdem eine dunkle Färbung, was im Gegensatz zu dem unmodifizierten Gel, das nahezu farblos ist, eine einfachere Handhabung nach visuellen Aspekten ermöglicht.

Gemäß einer bevorzugten Ausführung der vorliegenden Erfindung umfasst das hierin beschriebene Verfahren zum Entfernen von Nierensteinfragmenten folgenden weiteren Schritt, der zeitlich vor Schritt (ii) erfolgt:
Fragmentieren eines oder mehrerer Nierensteine in der Niere, so dass zwei oder mehrere, vorzugsweise eine Vielzahl von Nierensteinfragmenten entsteht bzw. entstehen.

Der beim Zertrümmern von Nierensteinen entstehende Gries lässt sich durch Anwendung der erfindungsgemäß anzuwendenden gelbildenden Zusammensetzung besonders effizient entfernen. Im Gegensatz zu den im Stand der Technik beschriebenen Techniken werden auch mittelgroße Fragmente während der Gelbildung zuverlässig erfasst und können idealerweise vollständig aus dem Körper entfernt werden.

Hierin beschrieben ist auch eine gelbildende Zusammensetzung, bestehend aus oder umfassend eine Zusammensetzung (A), enthaltend ein oder mehrere kationisch vernetzbare Polymere, und eine Zusammensetzung (B), enthaltend einen oder mehrere Vernetzungsmittel zum Vernetzen des bzw. der kationisch vernetzbaren Polymere, sowie zudem magnetisierbare Teilchen, wobei die magnetisierbaren Teilchen Bestandteil der Zusammensetzung (A) und/oder Bestandteil der Zusammensetzung (B) sind und/oder wobei die gelbildende Zusammensetzung zudem eine Zusammensetzung (C) umfasst, die magnetisierbare Teilchen enthält, zum teilweisen oder vollständigen Umschließen von Nierensteinen und/oder Nierensteinfragmenten in der Niere durch Ausbilden eines vernetzten Gels bei Kontakt der Zusammensetzung (A) mit der Zusammensetzung (B) (sowie gegebenenfalls zudem der Zusammensetzung (C)), wobei das vernetzte Gel magnetisierbare Teilchen enthält.

Die magnetisierbaren Teilchen können demnach auf beliebigem Weg in die gelbildende Zusammensetzung gelangen. Sie können sowohl Bestandteil einer oder beider der Zusammensetzungen (A) und/oder (B) sein oder aber auch als Bestandteil einer weiteren Zusammensetzung (C) zugegeben werden.

Gemäß einer bevorzugten Ausführung enthält die Zusammensetzung (A) und/oder die Zusammensetzung (B) (zudem) Chitosan. Besonders bevorzugt enthält die Zusammensetzung (B) Chitosan.

Gemäß einer (weiteren) bevorzugten Ausführung ist bzw. sind das bzw. ein, mehrere oder sämtliche kationisch vernetzbare Polymere der Zusammensetzung (A) ausgewählt aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine.

Hierbei gilt das oben zu den Polysacchariden der Zusammensetzung (A) bereits Gesagte entsprechend.

Gemäß einer weiteren bevorzugten Ausgestaltung d ist bzw. sind das bzw. ein, mehrere oder sämtliche Vernetzungsmittel der Zusammensetzung (B) ausgewählt aus der Gruppe bestehend aus zwei- und dreiwertigen Kationen, vorzugsweise Eisen- und Kalzium-Ionen.

Auch bezüglich der Vernetzungsmittel der Zusammensetzung (B) gilt das oben Gesagte entsprechend.

Gemäß einer weiteren bevorzugten Ausgestaltung der vorliegenden Erfindung weist die Zusammensetzung (B) einen sauren pH-Wert auf, vorzugsweise einen pH im Bereich von 3, 5 bis 4,5.

Auch hier gilt das oben zum pH-Wert einer hierin beschriebenen Zusammensetzung (B) Gesagte entsprechend.

Gemäß einer weiteren bevorzugten Ausführung der vorliegenden Erfindung sind die magnetisierbaren Teilchen ausgewählt aus Teilchen enthaltend oder bestehend aus ferromagnetische(n) Elemente(n) wie Eisen, Nickel und Kobalt sowie Legierungen wie AlNiCo, SmCo, Nd₂Fe₁₄B, Ni80Fe20, NiFeCo und/oder deren Oxide wie Eisenoxidpartikel, insbesondere Eisenoxidnanopartikel aus Fe₃O₄ und/oder γ-Fe₂O₃.

Wiederum gilt auch hier das oben zu den magnetisierbaren Teilchen Gesagte entsprechend.

Auch hierin beschrieben ist ein vernetztes Gel zum teilweisen oder vollständigen Umschließen von Nierensteinen und/oder Nierensteinfragmenten in der Niere, wobei das vernetzte Gel magnetisierbare Teilchen enthält und herstellbar oder hergestellt ist durch
(i) Bereitstellen einer Zusammensetzung (A), enthaltend ein oder mehrere kationisch vernetzbare Polymere, und einer Zusammensetzung (B), enthaltend einen oder mehrere Vernetzungsmittel zum Vernetzen des bzw. der kationisch vernetzbaren Polymere, sowie, optional, einer Zusammensetzung (C), wobei die Zusammensetzung (A) und/oder die Zusammensetzung (B) und/oder, falls vorhanden, die Zusammensetzung (C) magnetisierbare Teilchen enthält,
(ii) in-Kontakt-Bringen der Zusammensetzungen (A) und (B) (sowie gegebenenfalls (C)) unter Bedingungen, die eine Vernetzung des bzw. der kationisch vernetzbaren Polymere ermöglichen, so dass ein vernetztes Gel entsteht.

Die Herstellung eines solchen vernetzten Gels findet durch in-Kontakt-Bringen der Zusammensetzungen (A) und (B) (sowie gegebenenfalls (C)) statt. Das Abbinden erfolgt bevorzugt in einem Bereich der Niere, in dem Nierensteine und/oder Nierensteinfragmente, insbesondere von mittlerer Größe (vorzugsweise mit einem durchschnittlichen mittleren Durchmesser von 0.1 bis 4 mm, bevorzugt von 0.2 bis 3 mm, besonders bevorzugt von 0.5 bis 2 mm) vorliegen, damit diese vor Ort vollständig oder zumindest teilweise umschlossen werden. Das vernetzte Gel bindet vorzugsweise unter physiologischen Bedingungen ab und weist eine ausreichende Stabilität und Flexibilität auf, um vorzugsweise in einem Stück aus dem Körper extrahiert zu werden. Weitere bevorzugte Ausgestaltungen ergeben sich aus den vorangehenden Ausführungen.

Gemäß einer bevorzugten Ausführung des vernetzen Gels enthält die Zusammensetzung (A) und/oder die Zusammensetzung (B) Chitosan. Besonders bevorzugt enthält die Zusammensetzung (B) Chitosan.

Eine besonders bevorzugte Ausgestaltung betrifft ein vernetztes Gel wie oben beschrieben, wobei das bzw. ein, mehrere oder sämtliche kationisch vernetzbare Polymere der Zusammensetzung (A) ausgewählt ist bzw. sind aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine.

Hierbei gilt wiederum das oben zu den Polysacchariden der Zusammensetzung (A) bereits Gesagte entsprechend.

Eine weitere bevorzugte Ausgestaltung betrifft ein vernetztes Gel wie oben beschrieben, wobei das bzw. ein, mehrere oder sämtliche Vernetzungsmittel der Zusammensetzung (B) ausgewählt ist bzw. sind aus der Gruppe bestehend aus zwei- und dreiwertigen Kationen, vorzugsweise Eisen- und Kalzium-Ionen.

Auch in Bezug auf die Vernetzungsmittel der Zusammensetzung (B) gilt hier das oben Gesagte entsprechend.

Im Rahmen des vorliegenden Textes wird hierin auch ein Verfahren zum Entfernen von Nierensteinen und/oder Nierensteinfragmenten aus einem Bereich einer Niere, der zu entfernende Nierensteine und/oder Nierensteinfragmente enthält, beschrieben, bestehend aus oder umfassend folgende Schritte:
(i) Bereitstellen
   einer Zusammensetzung (A), enthaltend ein kationisch vernetzbares Polymer, vorzugsweise ein Polysaccharid mit deprotonierten Carboxy-Gruppen, besonders bevorzugt ein Polysaccharid ausgewählt aus der Gruppe der Polyuronide, insbesondere ein Alginat oder ein Pektin,
   und
   einer Zusammensetzung (B), enthaltend ein Vernetzungsmittel zum Vernetzen des bzw. der kationisch vernetzbaren Polymere, vorzugsweise zwei- und/oder dreiwertige Kationen, insbesondere Eisen- und/oder Kalzium-Ionen,
   wobei die Zusammensetzung (A) und/oder die Zusammensetzung (B) vorzugsweise zudem magnetisierbare Teilchen, zum Beispiel Eisenoxidpartikel, enthält,
   oder
   wobei zudem eine Zusammensetzung (C) bereitgestellt wird, die magnetisierbare Teilchen, zum Beispiel Eisenoxidnanopartikel, enthält,
(ii) gleichzeitiges oder zeitlich versetztes Einbringen der Zusammensetzungen (A) und (B) sowie gegebenenfalls (C) in den Bereich der Niere, der zu entfernende Nierensteine und/oder Nierensteinfragmente enthält, wobei die Zusammensetzung (B) vorzugsweise vor der Zusammensetzung (A) eingebracht wird, unter Bedingungen, die bei Kontakt der Zusammensetzung (A) mit der Zusammensetzung (B) (sowie gegebenenfalls der Zusammensetzung (C)) eine Vernetzung des bzw. der kationisch vernetzbaren Polymere ermöglichen, so dass ein vernetztes Gel entsteht, das die zu entfernenden Nierensteinfragmente teilweise oder vollständig umschließt und zudem magnetisierbare Teilchen enthält,
(iii) Entfernen des vernetzten Gels samt der davon umschlossenen Nierensteine und/oder Nierensteinfragmente aus der Niere.

Im Folgenden wird die vorliegende Erfindung anhand ausgewählter Beispiele näher erläutert.

### Kurze Beschreibung der Figuren:

Figur 1: Modifkationsstrategie zu CMC (Carboxymethylcellulose) mit DOPA und einer der unter R zusammengefassten Aminosäuren (unterer Kasten); die eingesetzten Aminosäuren sind zum Teil geschützt; von links nach rechts: 3,4-Dihydroxyphenylalanin, N-Boc-Lysin, t-Butyl-Cystein, Histidin.
Figur 2: Fotodokumentation einer Mischung aus katecholmodifizierter Carboxymethylcellulose (schwarzbraunes Hydrogel, oben) und des enzymfreien Referenzhydrogels (elfenbeinfarben, unten, hier grau dargestellt) auf Parafilm unter dem Einfluss der Schwerkraft im zeitlichen Verlauf.

### Beispiel 1: Herstellung von Zusammensetzungen (A), (B) und (C)

Zur Herstellung einer beispielhaften Zusammensetzung (A) werden 2 g Alginat in 200 mL Wasser gelöst.

Zur Herstellung einer beispielhaften Zusammensetzung (B) werden zunächst eine wässrige FeCl₃-Lösung (1 M), sowie eine wasserbasierte Chitosanlösung (0.32 Gew.-%, pH 6) und eine Oxalsäurelösung in Wasser (1 M) hergestellt. 3 mL der Chitosanlösung werden mit ca. 5 Tropfen der Oxalsäurelösung versetzt und zu diesem Gemisch werden 0.5 mL der Eisenchloridlösung gegeben.

Zur Herstellung einer beispielhaften Zusammensetzung (C) wird eine 4 bis 40 mM Eisen (0.35 bis 3.5 g pro Liter) enthaltende Partikel-Suspension in Wasser oder physiologischem Puffer zubereitet (M. Geppert et al., Nanotechnology 22 (2011) 145101). Diese Lösung wird zu 1 % bis 50% zu A oder B gegeben.

### Beispiel 2 (Versuch): Gelbildung mit modifizierten Biopolymeren am Beispiel eines Aminosäure Carboxymethylcellulose-Hybrids

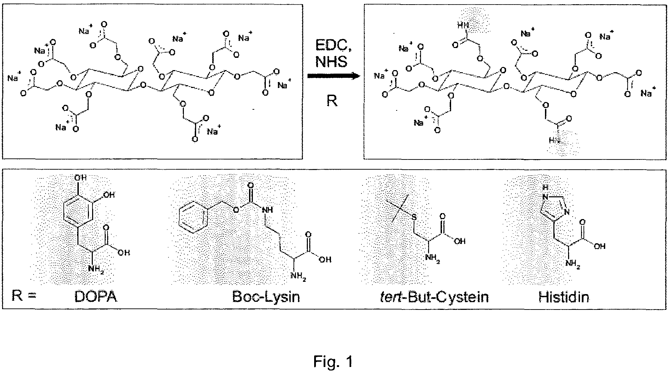

Das dem Alginat ähnliche Zuckerderivat Natrium-Carboxymethylcellulose (CMC) wurde jeweils mit den Aminosäuren DOPA, Lysin, Cystein und Histidin funktionalisiert (Figur 1). Das DOPA-modifizierte CMC wurde mit dem aminfunktionalen Polysaccharid Chitosan gemischt und das durch elektrostatische Interaktion zwischen den unterschiedlich geladenen Zuckern entstehende Hydrogel auf seine Adhäsionseigenschaften untersucht. Außerdem wurden sämtliche der Aminosäure-Cellulose-Hybride als Gelees angemischt und die Verbundfestigkeit bei der Adhäsion auf Titan überprüft. Ziel der Versuche war es, die adhäsiven Eigenschaften des (beispielhaften) Hybrids im feuchten Millieu zu verbessern, um die Einbindung der Nierensteine bzw. Nierensteinfragmente und die Flexibilität des Klebstoffes zu verändern.

Für die Funktionalisierung mit DOPA wurden zwei unterschiedliche Substitutionsgrade angestrebt. Jedes Mol CMC verfügt über acht Mol Acetatgruppen. Darauf bezogen wurden zum einen mit einem halben Promille (PA-S6) und zum anderen mit 0,3 Äquivalenten DOPA (PA-S7) modifiziert.

Die Natrium-Carboxymethylcellulose (CMC), wurde zunächst nach einem unkonventionellen Verfahren mit der Aminosäure 3,4-Dihydroxyphenylalanin (DOPA) funktionalisiert [1]. Hierfür wurden zunächst 2 g CMC (2 mmol) in 30 mL dd-Wassers über 90 min bei 40°C gelöst. Der pH der Lösung von ca. 7 wurde mit einer wässrigen HCl-Lösung (2 N) auf einen pH von 4-5 eingestellt. Zu der viskosen Lösung wurden 19 mg EDC (0,1 mmol) und 12 mg NHS (0,1 mmol) gegeben. Nach 30 min wurden 20 mg (0,1 mmol) DOPA, in 1,5 mL dd-Wasser gelöst, über eine feine Kanüle langsam, während die Lösung stetig rührte, hinzugetropft. Die Lösung wurde über Nacht weitergerührt.

Von dieser Lösung wurden 10 mL abgenommen (PA-S6). Weitere 2 mL der Lösung wurden für ATR Analysen lyophilisiert. Der verbleibende Rest (ca. 18 mL) der Lösung wurde mit 1,1 g EDC (5,8 mmol) und 0,7 g NHS (6,1 mmol) erneut 30 min aktiviert. Eine saure Lösung aus 0,6 g DOPA (3 mmol) in 10 mL dd-Wassers und 1 mL einer wässrigen HCl-Lösung (2N), wie schon für PA-S6, langsam über eine feine Kanüle zugegeben, während eine gute Durchmischung sichergestellt war. Von dieser Lösung PA-S7 wurde ebenfalls ein ATR angefertigt.

Das Produkt (PA-S6) wurde mit einer frischen 0,3 m%-igen Chitosanlösung (pH 6) zu gleichen Teilen gemischt. Die Mischung wurde auf zwei Gefäße aufgeteilt. Zu einer der beiden Mischungen wurden 0,5 v% einer frischen Laccaselösung (1 mg/mL) zugegeben. Beide Mischungen wurden gut durchmischt und im Anschluss, liegend auf einer beheizbaren Rüttelplatte, arretiert. Der Rüttler wurde auf eine Laufzeit von vier Stunden bei 47 °C und 650 rpm programmiert.

Die DOPA-CMC-Chitosan-Lösung (PAChi) und die DOPA-CMC-Chitosan-Lösung in Gegenwart der Peroxidase Laccase (PAChiLA), die die Vernetzung der Katechole initiiert, wurden inkubiert, um die Eigenschaften des erwarteten Hydrogels zu verändern. Dabei entstanden in beiden Reaktionsgefäßen elastische Hydrogele.

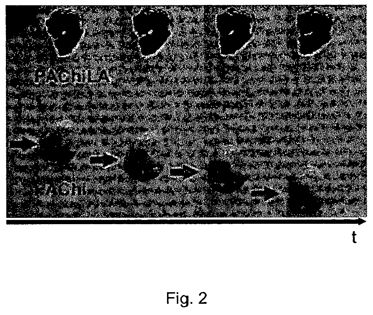

Die beiden Proben mit (PAChiLA) und ohne Laccase (PAChi) wurden danach hinsichtlich ihres unterschiedlichen Adhäsionspotentials untersucht. Hierfür wurden jeweils eine Probe (PAChiLA; PAChi) auf Parafilm aufgebracht und der Probenaufbau orthogonal ausgerichtet. Hierdurch wirkte die Schwerkraft auf die Haftfläche zwischen dem Cellulosehydrogel und der Parafilmoberfläche. Die Weglängen, die die Proben infolge der auf sie einwirkenden Schwerkraft über die Zeit zurücklegten wurden photographisch festgehalten (Figur 2).

Das Hydrogel der Referenz zeichnete sich durch eine elfenbeinähnliche Färbung aus, während das katecholhaltige Hydrogel, das durch Laccase polymerisiert wurde, braunschwarz gefärbt war. Diese Verfärbung ist charakteristisch für Polyphenole und spricht für eine Oxidation der DOPA-Gruppe am CMC-Polymerrückgrat. Durch die seitens des Chitosans angebotenen Amine, kann es zur Bildung eines kovalentes Netzwerkes infolge von Michaelreaktionen mit Aminen und zu radikalischer Addition gekommen sein. Die Bildung eines Hydrogels für die Referenz der katecholhaltigen Cellulose mit Chitosan ohne Enzym lässt sich mit elektrostatischer Wechselwirkung zwischen den noch freien Carbonsäuren der Carboxycellulose und den Aminen des Chitosans erklären. Beide Hydrogele wurden im Anschluss in einem Funktionsexperiment hinsichtlich ihrer makroskopischen Haftungsunterschiede auf Parafilm untersucht. Unter dem Einfluss des Kraftvektors Schwerkraft gaben die Wechselwirkungskräfte unterschiedlich stark nach, wie sich am zurückgelegten Weg ablesen lässt. Die Ergebnisse der Photodokumentation zeigen eine stärkere Haftung des vernetzten Hydrogels auf Parafilm im Vergleich mit der nicht kovalent vernetzten Referenz, die ohne Enzym inkubiert wurde.

Diese Ergebnisse bestärken die Annahme, daß es sich im Fall des oxidiert vorliegenden DOPA-CMC-Chitosan (PAChi) um kein kovalent vernetztes Hydrogel handelt. Der Zusammenhang zwischen Haftung und den durch das Katechol provozierten Veränderung in der Zuckermatrix, eröffnen steuerbare Hydrogeleigenschaften. Die rein elektrostatisch wechselwirkenden Netzwerke ordnen sich innerhalb des Hydrogels um und geben unter dem Einfluss eines Kraftvektors nach. Dieses Phänomen ist als Kriechverhalten für Thermoplasten bekannt.

Das enzymatisch oxidierte DOPA-CMC-Chitosan (PAChiLA) verfügt über kovalente Quervernetzungen innerhalb des Hydrogels und ist in einer Umorientierung infolge wirkender Scherkräfte eingeschränkt. Weiterhin kann es zu Wechselwirkungen zwischen den Polyphenolen im Hydrogelnetzwerk mit den Polyolefinen und Paraffinwachsen im Parafilm kommen.

Die Funktionalisierung der Carboxymethylcellulose wurde über das Katechol DOPA hinaus auf drei weitere Aminosäuren ausgeweitet (Figur 1). Ziel dieser Diversifizierung war es, durch Kombination aus diesen Hybriden, die adhäsiven Eigenschaften in feuchtem Milieu zu verbessern.

Für die Synthese wurden zunächst 13,5 g CMC (14 mmol) in einem 1 L Becherglas eingewogen und in 550 mL dd-Wasser unter leichtem Rühren bei 40 °C gelöst. Nach 90 Minuten wurde die klare gelbliche Lösung unter Rühren auf RT gekühlt. Wie oben beschrieben wurde das vorgelegte CMC unter Verwendung von EDC/NHS teils in N-Succinimid-Aktivester überführt. Nach ca. 40 Minuten wurde das Reaktionsgemisch auf fünf Erlmeyerkolben ä 83 mL (ca. 2 g CMC) aufgeteilt. Nun wurden jeweils 40 mmol der Aminosäure unter starkem Rühren in eines der Reaktionsgefäße gegeben.

76 mg DOPA bzw. 60 mg Histidin wurden zuvor jeweils in 100 µL HCl (2N) und 1900 µL dd-Wasser angelöst. 95 mg H-Lys(Boc) bzw. 83 H-Cys(tBut)-OH*HCl mussten in 2 mL dd-Wasser aufgenommen werden, um die Schutzgruppe zu erhalten. In Referenz wurde eine Probe ohne Zugabe einer Aminosäure mitgeführt. Nach 24 Stunden wurde die Reaktion abgebrochen und in Etappen jeweils 10 mL der Lösung lyophilisiert.

Auf eine Extraktion des Produktes gegen Ether wurde verzichtet, da die durch evtl. Verunreinigungen verursachten Fehler innerhalb des Fehlerbereichs des Funktionsexperimentes liegen. Unter dem Aspekt, dass dieses Experiment der Bewertung von adhäsiven Wechselwirkungen auf makroskopischer Ebene gewidmet ist, erschien dies plausibel. Die resultierenden Produkte (PX) wurden bei -20 °C gelagert. Die Nomenklatur ist in Tabelle 1 zusammengefasst.

**Tabelle 1: Nomenklatur der synthetisierten CMCs, PX; a) Mischung aus PH, PC, PA und PK (15:20:30:35).**

| Aminosäure | DOPA | Lysin | Cystein | Histidin | Mix^{a} | - |
|---|---|---|---|---|---|---|
| Aminosäure-CMC-Hybrid | PA | PK | PC | PH | PHCAK | P0 |

Die resultierenden Aminosäure-Cellulose-Hybride wurden jeweils als Gelees angemischt Zudem wurde eine Mischung aus den modifizierten CMCs aller vier Aminosäuren im Verhältnis PH : PC : PA : PK 15:20:30:35 verwendet (PHCAK). Diese Gelees wurden in eine Zellkulturplatte gespachtelt und einer Vorstudie zu den notwendigen Aushärtebedingungen unterzogen.

Basierend auf den Ergebnissen zu dieser Vorstudie wurden zwei Verbundfestigkeitsstudien zur Untersuchung der Adhäsion auf Titan angefertigt. In Studie A wurde das reine Gelee (P0) neben dem katecholhaltigen Gelee (PA) und dem cysteinmodifizierten Gelee (PC), sowie der Mischung PHCAKV unter Salzwasser auf Titan gefügt. Die Salzwasserlösung wurde, nachdem die Proben gefügt wurden, mit FeCl₃ als Oxidationsmittel versetzt.

In Studie B wurden Proben mit den gleichen Gelees gefügt. Zuvor wurden diese jedoch mit FeCl₃ vorbehandelt (geprimert), d.h. die Substrate wurden mit FeCl₃- Lösung benetzt und getrocknet. Danach wurde analog zu Studie A verfahren, jedoch ohne FeCl₃ der Salzwasserlösung zuzugeben.

Nach vier Tagen Auslagerung wurden die Proben geprüft, wobei die Prüflinge der Studie B sofortiges Adhäsionsversagen zeigten.

Die Prüflinge der Studie A wurden mit einem Bondtester in sechsfacher Bestimmung ausgewertet. Die Mischung PHCAK zeigte für die Adhäsionen ähnliche Festigkeiten wie die Prüfungen der Referenz (ca. 2 N). PA zeigte kaum noch messbare Adhäsion (ca. 1 N). Die Adhäsionsproben, die mit PC gefügt wurden, führten in allen Fällen zu Adhäsionsversagen und überstanden das Lösen aus der Fügevorrichtung nicht.

Die Bruchflächen zeigen in allen Fällen eine orangebraune Verfärbung der gehärteten Gelees. Diese Verfärbung tritt vor allem in den randnahen Bereichen auf. Weiterhin sind große Teile, die mit Gelee benetzt waren, nicht verfärbt und lagen noch Gel-artig in ihrer Konsistenz vor. Die Proben, die den Scherkräften am längsten standhielten, weisen in großen Teilen des benetzten Bereiches Adhäsionsbrüche auf. Kohäsionsbrüche zeigen sich nur vereinzelt (ca. 5 %) an den Rändern.

Das vorzeitige Adhäsionsversagen der PC-Proben läßt sich durch das Fehlen möglicher Reaktionspartner wie Katecholen erklären. Dennoch bleibt offen, warum diese Fügungen, als auch die von PA schlechter adhärierten als die Referenz.

Die geprüften Adhäsivproben präsentierten in den Bruchflächen klare Indizien für die Ursache der nachgiebigen Fügung. Nur am Rand befinden sich rigide Bereiche, die in Einzelfällen als Zeugen eines Kohäsionsbruchs zu deuten sind. Die rotbraune Verfärbung wird durch die komplexierten Eisenionen verursacht. Die Bereiche, die sich weiter entfernt vom Rand befinden, liegen sichtbar noch als Gel vor. Diese Bereiche leisten demnach keinen Kohäsionsbeitrag zur Verbundfestigkeit.

Dies äußerte sich ebenfalls im Verlauf des Kraft-Zeit-Diagramms der Scherprüfung (nicht gezeigt). Aus der parabelartigen Funktion konnte abgelesen werden, dass es sich um ein nicht gehärtetes Adhäsiv handelte. Dieser Effekt wurde auch von Cha et al. bestätigt [2]. Sie hatten ein Muschelprotein über Bakterien exprimiert und die posttranslationale Modifikation des Tyrosins im Kolben vorgenommen. Die Adhäsivfügungen mit unmodifizierten Protein zeigten einen ähnlich parabelartigen Verlauf.

Die katecholhaltigen Proben (nach posttranslationaler Oxidation durch Tyrosinase) indes gaben die typische Kurve aus, in der ein Bindungsbruch an einem rapiden Abfall der Kraft, die gegen die angelegte Scherkraft wirkt, festzumachen ist.

Eine weitergehende Betrachtung der Bruchflächen legt offen, weshalb diese Fügung nicht homogen härten konnte. Die Härtung ist ähnlich wie bei einem Polyurethan diffusionskontrolliert. Ab einer kritischen Schichtdicke, in diesem Fall der Abstand zum Rand, stoppt die Härtung aufgrund der Abwesenheit der notwendigen Eisenionen. Diese Abhängigkeit der Härtung durch Komplexbildung wurde in einem korrespondierenden Experiment, der Hydrogelstudie, plakativ untersucht. Da in diesem Fall keine Fügeteile die Gelees begrenzen, konnte der Härtungsfortschritt photographisch dokumentiert werden. Das Ergebnis der Probe für die Mischung PHCAK zeigte von allen Proben den homogensten Härtungsprozess. Die Ergebnisse der korrespondierenden Auslagerung der Gelpellets in Salzwasser, das mit FeCl₃-Lösung versetzt wurde, erbrachte für die Pellets von PHCAK wie auch in der Studie A zur Verbundfestigkeit die homogensten Härtungsfortschritte.
[1] Leung, A. C. W.; Hrapovic, S.; Lam, E.; Liu, Y.; Male, K. B.; Mahmoud, K. A.; Luong, J. H. T. small 2011, 7, 302-305.
[2] Cha, H. J.; Hwang, D. S.; Lim, S.; White, J. D.; Matos-Perez, C. R.; Wilker, J. J. Biofouling 2009, 25, 99-107.

### Beispiel 3: Anwendung einer erfindungsgemäß anzuwendenden gelbildenden Zusammensetzung

Ein Katheter wird durch den Harntrakt in einen Bereich der Niere, der zu entfernende Nierensteinfragmente enthält, eingebracht. 50 mL einer Zusammensetzung (B) gemäß Beispiel 1 werden durch den Katheter in den Bereich der Niere eingeleitet. Im Anschluss wird eine Zusammensetzung (A) gemäß Beispiel 1 durch den oder einen weiteren Katheter in den Bereich der Niere eingeleitet, wodurch es über einen Zeitraum von ca. 1 min zur Gelbildung kommt. Dann wird ein endoskopisches Greifinstrument über den Harntrakt eingeführt. Mit dem Greifinstrument wird das gefestigte Gel gefasst und durch Extraktion über den Harntrakt aus dem Körper entfernt.

## Patentansprüche

1. Gelbildende Zusammensetzung, umfassend eine Zusammensetzung (A), enthaltend ein oder mehrere kationisch vernetzbare Polymere, und eine Zusammensetzung (B), enthaltend ein oder mehrere Vernetzungsmittel zum Vernetzen des bzw. der kationisch vernetzbaren Polymere zur Anwendung in einem Verfahren zur Einbettung und zum Entfernen von Nierensteinen und/oder Nierensteinfragmenten in/aus einem Bereich einer Niere, der zu entfernende Nierensteine und/oder Nierensteinfragmente enthält, mit folgenden Schritten:
(i) Bereitstellen der Zusammensetzungen (A) und (B),
(ii) Einbringen der Zusammensetzungen (A) und (B) in einen Bereich der Niere, der zu entfernende Nierensteine und/oder Nierensteinfragmente enthält,
unter Bedingungen, die bei Kontakt der Zusammensetzung (A) mit der Zusammensetzung (B) eine Vernetzung des bzw. der kationisch vernetzbaren Polymere ermöglichen, so dass ein vernetztes Gel entsteht, das die zu entfernenden Nierensteine und/oder Nierensteinfragmente teilweise oder vollständig umschließt, wodurch die Entfernung der eingebetteten zu entfernenden Nierensteine und/oder Nierensteinfragmente ermöglicht wird,
(iii) Entfernen des vernetzten Gels samt der davon umschlossenen Nierensteine und/oder Nierensteinfragmente aus der Niere.

2. Gelbildende Zusammensetzung zur Anwendung nach Anspruch 1, wobei das eine bzw. die mehreren oder sämtliche kationisch vernetzbare(n) Polymer(e) der Zusammensetzung (A) ausgewählt ist bzw. sind aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate , Pektine und Natrium-Carboxymethylcellulose (CMC) funktionalisiert mit 3,4-Dihydroxyphenylalanin (DOPA).

3. Gelbildende Zusammensetzung zur Anwendung nach Anspruch 1 oder Anspruch 2, wobei das eine bzw. die mehreren oder sämtliche Vernetzungsmittel der Zusammensetzung (B) ausgewählt ist bzw. sind aus der Gruppe bestehend aus zwei- und dreiwertigen Kationen, vorzugsweise Eisen- und Kalzium-Ionen.

4. Gelbildende Zusammensetzung zur Anwendung nach einem der vorangehenden Ansprüche, wobei die Zusammensetzung (B) einen sauren pH-Wert aufweist, vorzugsweise einen pH im Bereich von 3,5 bis 4,5.

5. Gelbildende Zusammensetzung zur Anwendung nach einem der vorangehenden Ansprüche, wobei die gelbildende Zusammensetzung zudem magnetisierbare Teilchen enthält, wobei
- die magnetisierbaren Teilchen Bestandteil der Zusammensetzung (A) und/oder Bestandteil der Zusammensetzung (B) sind
und/oder
- die gelbildende Zusammensetzung zudem eine Zusammensetzung (C) umfasst, die magnetisierbare Teilchen enthält, wobei dann in Schritt (i) neben den Zusammensetzungen (A) und (B) auch die Zusammensetzung (C) bereitgestellt und in Schritt (ii) zeitversetzt oder gleichzeitig mit der Zusammensetzung (A) oder der Zusammensetzung (B) auch die Zusammensetzung (C) eingebracht wird,
so dass das vernetzte Gel zudem magnetisierbare Teilchen enthält.

6. Gelbildende Zusammensetzung zur Anwendung nach einem der vorangehenden Ansprüche, wobei die magnetisierbaren Teilchen ausgewählt sind aus Teilchen enthaltend oder bestehend aus ferromagnetische(n) Elemente(n) wie Eisen, Nickel und Kobalt sowie Legierungen wie AlNiCo, SmCo, Nd₂Fe₁₄B, Ni80Fe20, NiFeCo und/oder deren Oxide wie Eisenoxidpartikel, insbesondere Eisenoxidnanopartikel aus Fe₃O₄ und/oder γ-Fe₂O₃.

7. Gelbildende Zusammensetzung zur Anwendung nach einem der vorangehenden Ansprüche, wobei die Zusammensetzung (A) und/oder die Zusammensetzung (B) zusätzlich Chitosan enthält, bevorzugt, wobei die Zusammensetzung (B) Chitosan enthält.

8. Gelbildende Zusammensetzung zur Anwendung nach einem der vorangehenden Ansprüche, wobei in Schritt (ii) die Zusammensetzungen (A) und (B) nacheinander oder gemeinsam eingebracht werden.

9. Gelbildende Zusammensetzung zur Anwendung nach einem der vorangehenden Ansprüche, wobei in Schritt (ii) die Zusammensetzungen (B) vor der Zusammensetzung (A) eingebracht wird.

10. Gelbildende Zusammensetzung zur Anwendung nach einem der vorangehenden Ansprüche, wobei das Verfahren folgenden weiteren Schritt umfasst, der zeitlich vor Schritt (ii) erfolgt:
Fragmentieren eines oder mehrerer Nierensteins/e in der Niere, so dass zwei oder mehrere, vorzugsweise eine Vielzahl von Nierensteinfragmenten entstehen.

## Claims

1. Gel-forming system comprising a composition (A), comprising one or several cationically crosslinkable polymer(s), and a composition (B), comprising one or several crosslinking agent(s) for crosslinking the cationically crosslinkable polymer(s),
for use in a method for embedding or removing kidney stones and/or fragments thereof from a region of a kidney that contains kidney stones and/or fragments thereof, that are to be removed, with the following steps:
(i) providing the compositions (A) and (B),
(ii) introducing the compositions (A) and (B) into a region of the kidney that contains kidney stones and/or fragments thereof that are to be removed,
under conditions enabling crosslinking of the cationically crosslinkable polymer(s) upon contact of composition (A) with composition (B), so that a crosslinked gel is formed that partly or fully surrounds the kidney stones and/or fragments thereof, that are to be removed, by which the removal of the embedded kidney stones and/or fragments thereof, that are to be removed, is enabled,
(iii) removing the crosslinked gel together with the kidney stones and/or fragments thereof that are surrounded by it from the kidney.

2. Gel-forming system for use according to claim 1, wherein the or one, several or all of the cationically crosslinkable polymer(s), respectively, of compound (A) is or are selected from the group consisting of polysaccharides, more particularly polysaccharides with deprotonated or deprotonatable functional groups, preferably carboxy groups, preferably polysaccharides from the group of polyuronides, particularly preferred polysaccharides from the group of alginates, pectins and sodium carboxymethyl cellulose (CMC) functionalized with 3,4-dihydroxyphenylalanine (DOPA).

3. Gel-forming system for use according to claim 1 or claim 2, wherein the or one, several or all of the crosslinking agent(s), respectively, of compound (B) is or are selected from the group consisting of divalent and trivalent cations, preferably iron and calcium ions.

4. Gel-forming system for use according to any of the preceding claims, wherein composition (B) has an acidic pH-value, preferably a pH in the range of 3.5 to 4.5.

5. Gel-forming system for use according to any of the preceding claims, wherein the gel-forming system in addition contains magnetizable particles, wherein
- the magnetizable particles are part of composition (A) and/or composition (B)
and/or
- the gel-forming system in addition comprises a composition (C) that contains magnetizable particles, wherein then in step (i) composition (C) is also provided besides compositions (A) and (B) and in step (ii) composition (C) is introduced as well, time-delayedly or at the same time, with composition (A) or composition (B),
so that the crosslinked gel additionally contains magnetizable particles.

6. Gel-forming system for use according to any of the preceding claims, wherein the magnetizable particles are selected from particles comprising or consisting of ferromagnetic elements such as iron, nickel and cobalt as well as alloys such as AlNiCo, SmCo, Nd₂Fe₁₄B, Ni₈₀Fe₂₀, NiFeCo and/or oxides thereof such as iron oxide particles, more particularly iron oxide nanoparticles made of Fe₃O₄ and/or γ-Fe₂O₃.

7. Gel-forming system for use according to any of the preceding claims, wherein composition (A) and/or composition (B) additionally contain chitosan, preferably, whereby composition (B) contains chitosan.

8. Gel-forming system for use according to any of the preceding claims, wherein in step (ii) the compositions (A) and (B) are introduced successively or together.

9. Gel-forming system for use according to any of the preceding claims, wherein in step (ii) composition (B) is introduced before composition (A).

10. Gel-forming system for use according to any of the preceding claims, wherein the method comprises the following step that takes place in terms of time before step (ii):
Fragmentation of one or several kidney stone(s) in the kidney, so that two or several, preferably a plurality of kidney stone fragments are formed.

## Revendications

1. Composition gélifiante comprenant une composition (A) contenant un ou plusieurs polymères réticulables par voie cationique et une composition (B) contenant un ou plusieurs agents de réticulation destinés à réticuler le ou bien les polymères réticulables par voie cationique, pour l'application dans un procédé destiné à enrober et à éliminer des calculs rénaux et/ou des fragments de calcul rénal dans une/d'une zone d'un rein laquelle contient des calculs rénaux et/ou fragments de calcul rénal à éliminer, comprenant les étapes suivantes:
(i) fournir les compositions (A) et (B),
(ii) introduire les compositions (A) et (B) dans une zone du rein laquelle contient des calculs rénaux et/ou fragments de calcul rénal à éliminer, dans des conditions qui, lors d'un contact de la composition (A) avec la composition (B), permettent une réticulation du ou bien des polymères réticulables par voie cationique de sorte qu'un gel réticulé est formé qui enveloppe partiellement ou complètement les calculs rénaux et/ou fragments de calcul rénal à éliminer, permettant ainsi l'élimination des calculs rénaux et/ou fragments de calcul rénal enrobés à éliminer,
(iii) éliminer du rein le gel réticulé avec les calculs rénaux et/ou fragments de calcul rénal enveloppés par celui-ci.

2. Composition gélifiante pour l'application selon la revendication 1, dans laquelle ledit un ou bien lesdits plusieurs ou tous les polymère(s) réticulable(s) par voie cationique de la composition (A) est ou bien sont choisi(s) dans le groupe constitué par les polysaccharides, en particulier les polysaccharides comprenant des groupes fonctionnels déprotonés ou aptes à être déprotonés, de préférence des groupes carboxyles, de préférence les polysaccharides provenant du groupe des polyuronides, de manière particulièrement préférée les polysaccharides provenant du groupe des alginates, des pectines et de la carboxyméthylcellulose (CMC) de sodium fonctionnalisée avec la 3,4-dihydroxyphénylalanine (DOPA).

3. Composition gélifiante pour l'application selon la revendication 1 ou la revendication 2, dans laquelle ledit un ou bien lesdits plusieurs ou tous les agent(s) de réticulation de la composition (B) est ou bien sont choisi(s) dans le groupe constitué par des cations bi- et trivalents, de préférence des ions de fer et de calcium.

4. Composition gélifiante pour l'application selon l'une quelconque des revendications précédentes, dans laquelle la composition (B) présente une valeur pH acide, de préférence une valeur pH comprise entre 3,5 et 4,5.

5. Composition gélifiante pour l'application selon l'une quelconque des revendications précédentes, la composition gélifiante contenant en outre des particules magnétisables, dans laquelle
- les particules magnétisables entrent dans la composition de la composition (A) et/ou entrent dans la composition de la composition (B), et/ou
- la composition gélifiante comprend en outre une composition (C) qui contient des particules magnétisables, à l'étape (i) étant fournie, outre les compositions (A) et (B), également la composition (C), et à l'étape (ii) étant introduite également la composition (C) soit de manière décalée dans le temps soit simultanément avec la composition (A) ou la composition (B),
de sorte que le gel réticulé contient en outre des particules magnétisables.

6. Composition gélifiante pour l'application selon l'une quelconque des revendications précédentes, dans laquelle les particules magnétisables sont choisies parmi des particules comprenant ou constituées par des éléments ferromagnétiques tels que le fer, le nickel et le cobalt ainsi que des alliages tels que AlNiCo, SmCo, Nd₂Fe₁₄B, Ni80Fe20, NiFeCo et/ou des oxydes de ceux-ci, tels que les particules d'oxyde de fer, en particulier les nanoparticules d'oxyde de fer en Fe₃O₄ et/ou γ-Fe₂O₃.

7. Composition gélifiante pour l'application selon l'une quelconque des revendications précédentes, dans laquelle la composition (A) et/ou la composition (B) contient en plus le chitosane, de préférence la composition (B) contenant le chitosane.

8. Composition gélifiante pour l'application selon l'une quelconque des revendications précédentes, dans laquelle, à l'étape (ii), les compositions (A) et (B) sont introduites l'une après l'autre ou en commun.

9. Composition gélifiante pour l'application selon l'une quelconque des revendications précédentes, dans laquelle, à l'étape (ii), la composition (B) est introduite avant la composition (A).

10. Composition gélifiante pour l'application selon l'une quelconque des revendications précédentes, le procédé comprenant l'autre étape suivante qui est réalisée temporellement avant l'étape (ii):
fragmenter un ou plusieurs calcul(s) rénal/rénaux dans le rein de sorte que deux ou plusieurs, de préférence une pluralité de fragments de calcul rénal sont formés.
